# EUROPEAN PATENT APPLICATION

(11) **EP 2 634 258 A1**
(43) Date of publication of application: **04.09.2013**
(21) Application number: 12157576.5
(22) Date of filing: 29.02.2012
(51) Int. Cl.: C12P 21/02, C12N 9/48

(54) **Enzymatic synthesis of amino acid and peptide c-terminal amides**

(71) Applicant: Enzypep B.V., 6167 RD Geleen (NL)
(72) Inventor: Nuijens, Timo, 6100 AA Echt (NL); Quaedflieg, Peter Jan Leonard Mario, 6100 AA Echt (NL)
(74) Representative: Jansen, Cornelis Marinus

(57) **Abstract**

Method for enzymatically synthesising an amino acid or peptide *C-*terminal amide, from the corresponding N-protected amino acid ammonium salt or optionally N-protected peptide *C*-terminal carboxylic acid ammonium salt, by contacting said N-protected amino acid ammonium salt or optionally N-protected peptide C-terminal carboxylic acid ammonium salt with a serine endopeptidase. in an organic solvent or an organic solvent mixture containing 1 vol% or less water.

## Description

The invention relates to a method for enzymatically preparing a *C-*terminal amide, from the C-terminal carboxylic acid ammonium salt of an *N*-protected amino acid or an optionally *N*-protected peptide.

For the purpose of this invention, with an amide is meant a -C(=O)-NH₂ group, with a carboxylic acid is meant a -C(=O)-OH group and with a carboxylic ammonium salt is meant a -C(=O)-O-NH₄⁺ salt.

For the purpose of this invention with amino acid or peptide *C-*terminal amide is meant that the (*C*-terminal) amino acid residue contains an α-amide functionality.

For the purpose of this invention, with a direct ammonia source is meant the addition of NH₃ as a gas or in the solubilized form.

For the purpose of this invention, with an external ammonia source is meant the addition of ammonia in the form of an ammonium salt (NH₄⁺X⁻, with X being any suitable counter ion, organic or inorganic).

For the purpose of this invention, with amino acid or peptide ammonium salt is meant that the *C*-terminal carboxylic acid functionality, and optionally the side-chain carboxylic acid functionalities, have an ammonium ion as counter ion.

The C-terminal amide functionality of peptides is important for their biological activity, but also increases their stability against exoprotease activity (see, *e.g*., Wollack et al., J. Am. Chem. Soc. 2009, 131, 7293-7303). Examples of commercial pharmaceutical peptides containing a *C*-terminal amide functionality are triptorelin, cetrorelix, calcitonin, lanreotide, ganirelix, exenatide, pramlintide, desmopressin and nafarelin.

Solution phase synthesis of peptide *C*-terminal amides usually starts from the *C*-terminal amino acid carboxamide and the desired peptide sequence is stepwise assembled in the *C* → *N* direction using *N*-carbamate protected amino acid building blocks to minimise racemisation. However, the generally limited solubility of amino acid and peptide *C*-terminal amides make the synthesis of longer peptide amides rather laborious by solution phase methodology. Therefore, peptide amides are mostly synthesized by solid phase peptide synthesis (SPPS) using, among others, Rink Amide resin or 4-methylbenzhydrylamine (MBHA) resin in Fmoc- or Boc-SPPS, respectively. However, these resins are expensive and not attractive for large scale use. On the other hand the solid-phase synthesis of peptide *C*-terminal carboxylic acids is much more cost-efficient since cheap and industrially available resins can be used (such as the 2-chlorotritylchloride resin). Alternatively, several peptide *C*-terminal carboxylic acids are accessible via fermentative routes. Therefore, several chemical approaches have been developed to convert peptide *C*-terminal carboxylic acids into the corresponding amides. Unfortunately, these methods lack selectivity toward the *C-*terminus and usually cause racemisation or prove incompatible with labile substrates.

In contrast to chemical approaches for modification of amino acids and peptides, enzymatic conversions proceed under mild conditions and are often chemo-, regio- and stereoselective. For instance, in nature, peptide amidases hydrolyse peptide *C*-terminal amides, which is a reversable type of reaction. The peptide amidase from the *flavedo* of oranges (PAF) has been described in the literature for the synthesis of peptide C-terminal amides using ammonium hydrogen carbonate as external ammonium source, but PAF (and other peptide amidases) tend to be very selective for a limited number of amino acids and are not accessible on large scale (*e.g*. described by 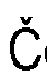 e 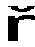 ovsk 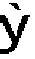 et al. *Angew. Chem. Int. Ed.* 1998, *37,* 1885-1887; Biotechnol. Appl. Biochem. 2001, 33, 181-187). Furtheremore, amidation yields are very low (< 38%).

Other enzymes than peptide amidases have been described for amide synthesis starting from an amino acid or peptide alkyl ester. Reactions tend to be fast and high yielding, although it should be taken into account that there is an optimum in product yield because both the starting material alkyl ester and the amide product can be hydrolysed by the enzyme of use, leading to side compounds. Lipases, such as *Candida antarctica* lipase-B (Cal-B), have been described to perform esterification and subsequent amidation of a number of aliphatic carboxylic acids and D-phenylglycine (*e.g*. described by Litjens et al. Chem. Commun. 1999, 1255-1256). However, the direct amidation of amino acid and peptide *C*-terminal carboxylic acids by lipases (such as Cal-B) has so far not been reported. *C*-terminal amidation of amino acid and peptide *C*-terminal esters has been described using the protease Subtilisin A (Chen et al. Synthesis 1993, 858-860) and with ammonium chloride as ammonium source. This amidation reaction has very recently been optimized using *C*-terminal peptide methyl esters and ammonium carbamate as amine source (Boeriu et al. J. Mol. Cat. B. Enzymatic. 2010, 66, 33-42). However, the synthesis of the *C*-terminal peptide (methyl) esters that are required for the enzymatic reaction encounters almost the same disadvantages as the direct chemical amidation, *i.e*. lack of *C*-terminal selectivity, racemisation issues, and incompatibility with labile substrates or other protecting groups.

Very recently, one example was given for the amidation of a dipeptide C-terminal carboxylic acid using Subtilisin A and 10 equivalents of ammonium carbamate as ammonium source (US patent application # 20090053760, example 93) in organic solvents. The maximum yield obtained for the reaction (Cbz-Ala-Phe-OH to Cbz-Ala-Phe-NH₂) was 69% with a reaction time of 21 h as compared to 87% in 21 h when starting from the methyl ester, *i.e*. Cbz-Phe-Ala-OMe.

The inventors surprisingly found that the amide bond formation using the *C*-terminal ammonium salt of the amino acid or peptide itself (instead of an external ammonium salt as ammonia source), can be performed in much higher yields and with shorter reaction times using a serine endopeptidase in nearly anhydrous organic solvents. Within the enzyme classification system such serine endopeptidases fall within class E.C. 3.4.21.

Accordingly, the present invention relates to a method for enzymatically synthesising an amino acid or peptide *C*-terminal amide, from the corresponding amino acid or peptide *C*-terminal carboxylic acid ammonium salt, in an organic solvent or an organic solvent mixture containing 1 vol% of water or less, by a serine endopeptidase.

The invention has the advantage that only one equivalent of ammonia is needed and that downstream processing and purification is much easier because there are no external ammonium salts (NH₄⁺X⁻, with X being any suitable counter ion, organic or inorganic) and related side compounds (HX) which have to be separated from the product.

The invention has the further advantage that no or very little peptide transamidation or peptide hydrolysis in the peptide chains occurs.

In addition, the method of the invention allows the synthesis of *C-*terminal amides of amino acids and peptides bearing protected side-chain functionalities as well as of amino acids and peptides bearing unprotected side-chain functionalities. Such side-chain functionalities which may be protected or unprotected are in particular side groups selected from thiol groups, hydroxyl groups, primary amine groups, carboxyl groups, carboxyamide groups, guanidino groups, imidazole groups, indole groups and thioether groups. It is particularly surprising that peptides containing one or more side chain protected amino acid residues, are still recognized by the serine endopeptidase.

In addition, the method of the invention allows the synthesis of the *C-*terminal amide of peptides having a protected *N*-terminal amine as well as peptides having an unprotected *N*-terminal amine group.

As used herein, the term amino acid includes proteinogenic and non-proteinogenic amino acids. Likewise, as used herein, the term peptides includes peptides composed of proteinogenic amino acids, non-proteinogenic amino acids and combinations thereof. Proteinogenic amino acids are the amino acids that may be found in proteins and that are coded for by the standard genetic code. The proteinogenic amino acids are glycine, L-alanine, L-valine, L-leucine, L-isoleucine, L-serine, L-threonine, L-methionine, L-cysteine, L-asparagine, L-glutamine, L-tyrosine, L-tryptophan, L-aspartic acid, L-glutamic acid, L-histidine, L-lysine, L-arginine, L-proline and L-phenylalanine.

Examples of non-proteinogenic amino acids include D-enantiomers of proteinogenic amino acids, D-phenylglycine, L-phenylglycine, D-4-fluoro-phenylalanine and L-4-fluoro-phenylalanine.

The ammonium salt of the substrate which is reacted may in particular be represented by a compound of formula (I).

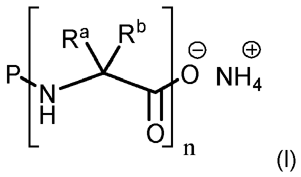

Herein P stands for a hydrogen or *N*-terminal protecting group. Suitable *N*-terminal protecting groups are those *N*-protecting groups which can be used for the synthesis of (oligo)peptides. Such groups are known to the person skilled in the art. Examples of suitable *N*-protecting groups include carbamate or acyl type protecting groups, for instance 'Cbz' (benzyloxycarbonyl), 'Boc' (tert-butyloxycarbonyl), 'For' (formyl), 'Fmoc' (9-fluorenylmethoxycarbonyl), 'PhAc' (phenacetyl) and 'Ac' (acetyl). The groups For, PhAc and Ac may be introduced and cleaved enzymatically using the enzymes Peptide Deformylase, PenG acylase or Acylase, respectively. Chemical cleavage methods are generally known in the art. In particular, in case n = 1, the N-protective group is generally needed to allow the amidation to proceed well.

Herein, n is an integer of at least 1. n May in particular be at least 2, at least 3, at least 4, at least 5, at least 6, at least 7, at least 8, at least 9 or at least 10. n May in particular be 100 or less, 75 or less, 50 or less, 25 or less, 20 or less 15 or less, e.g. 10 or less.

Herein, each R^{a} and each R^{b} independently represent a hydrogen atom or an organic moiety, preferably an amino acid side chain. Thus, it is not required that R^{A} is the same in all n amino acid units. Similarly, it is not required that R^{B} is the same in all n amino acid units. Preferably, for each amino acid residue as depicted between the square brackets [ and ] in formula (I), at least one of R^{a} and R^{b} is H R^{A} and/or R^{B} in the compound of formula I according to the invention may contain a functional group e.g. hydroxyl, carboxylic acid, primary or secondary amine (including e.g. indole and guanidino), thiol or primary amide functionalities i.e. have a side-chain functionality. The functional groups present in R^{A} and R^{B} may have been protected with protecting groups known in the art. Many different protecting groups are known, and can be used in the method according to the invention. If Fmoc based solid-phase peptide synthesis is used, the side-chain protecting groups can for instance be selected from the t-Bu (tert-butyl), Boc, Trt (trityl), Mtt (4-methyltrityl), Acm (acetamidomethyl), Dnp (2,4-dinitrophenyl), Pmc (2,2,5,7,8-pentamethylchroman-6-sulfonyl) or Pbf (2,2,4,6,7-pentamethyldihydrobenzofuran-5-sulfonyl) groups. If Boc based solid-phase peptide synthesisis is used, the side-chain protecting groups can for instance be selected from the Bzl (benzyl), Bz (benzoyl), 2Cl-Z (2-chlorobenzyloxycarbonyl), cHex (cyclohexyl), Tos (tosyl), Xan (xanthenyl), For or Mbzl (4-methoxybenzyl) and 3-benzyloxymethyl groups.

In the context of the invention with 'amino acid side-chain' is meant any proteinogenic or non-proteinogenic amino acid side-chain. The carboxylic acid side-chain functionalities of e.g. aspartic and glutamic acid may also be in the form of an ammonium salt.

In principle, the ammonium salt of the amino acid or peptide can be prepared *in situ* by the addition of NH₃ as a gas or in the solubilized form *e.g*. ammonia in methanol, ethanol, isopropanol or dioxane, to a solution or suspension of the amino acid or peptide *C*-terminal acid. Alternatively, the ammonium salt of the amino acid or peptide can be prepared seperately followed by exposure to a serine endopeptidase in an organic solvent or organic solvent mixture..

In the method of the invention the C-terminal amidation is catalysed by a serine endopeptidase (E.C. 3.4.21). When referring to a serine endopeptidase from a particular source, recombinant serine endopeptidases originating from a first organism, but actually produced in a (genetically modified) second organism, are specifically meant to be included as enzymes from that first organism.

Preferably, the serine endopeptidase used in the method of the invention is a subtilisin. In the enzyme classification system the class for subtilisins is E.C.3.4.21.62.

Various subtilisins are known in the art, see e.g. US 5,316,935 and the references cited therein. Such subtilisins may be used in the method according to the invention.

Examples of organisms from which a subtilisin used in the method of the invention may be derived include Trichoderma species, such as from Trichoderma reesei; Rhizopus species, such as from Rhizopus oryzae; Bacillus species, such as from Bacillus licheniformis, Bacillus subtilis, Bacillus amyloliquefaciens, Bacillus clausii, Bacillus lentus, Bacillus alkalophilus, Bacillus halodurans; Aspergillus species, such as from Aspergillus oryzae or Aspergillus niger; Streptomyces species, such as from Streptomyces caespitosus or Streptomyces griseus; Candida species; fungi; Humicola species; Rhizoctonia species; Cytophagia; Mucor species; and animal tissue, in particular from pancreas, such as from porcine pancreas, bovine pancreas or sheep pancreas.

It will be clear to the average person skilled in the art that use can also be made of mutants of naturally occurring (wild type) subtilisins in a method according to the invention. Mutants of wild-type enzymes can for example be made by modifying the DNA encoding the wild-type enzymes using mutagenesis techniques known to the person skilled in the art (random mutagenesis, site-directed mutagenesis, directed evolution, gene shuffling, etc.) so that the DNA encodes an enzyme that differs by at least one amino acid from the wild-type enzyme or so that it encodes an enzyme that is shorter compared to the wild-type and by effecting the expression of the thus modified DNA in a suitable (host) cell. Mutants of the enzyme may have improved properties, for instance with respect to one or more of the following aspects: substrate scope, activity, stability, organic solvent resistance, temperature profile and side reaction profile.

In a preferred method subtilisin A is used to catalyse the amidation reaction. Subtilisin A is a commercially available subtilisin from Novozymes and has been found particularly advantageous with respect to give the desired peptide C-terminal amide product with a good yield in a relatively short time.

Alcalase® is a suitable source for subtilisin A. This product is available from Novozymes (Bagsvaerd, Denmark). Alcalase® is a cheap and industrially available proteolytic enzyme mixture produced by *Bacillus licheniformis* (containing subtilisin A as a major enzyme component).

Commercially available enzymes, such as Alcalase®, may be provided by the supplier as a liquid, in particular an aqueous liquid. In such case, the enzyme is preferably first isolated from undesired liquid, for instance excess water or alcohols that cause an undesired side-reaction. This may suitably be accomplished by precipitation, usually followed by separation of the solid from the liquid, and/or drying. Precipitation may be accomplished using an alcohol, such as t-butanol. In case another alcohol is used, care should be taken that such alcohol does not interfere adversely with the amidation reaction.

In a preferred embodiment, the enzyme is used in an immobilized form. At least in some embodiments this may result in an increased yield of synthesised peptide *C*-terminal amide after a relatively short reaction time. Particularly good results have been obtained with Alcalase cross-linked enzyme aggregates (Alcalase-CLEAs) or with Alcalase immobilized on solid particles such as Alcalase-Imibond, Alcalase-Epobond, Alcalase-immozyme or Alcalase-Decalite. Immobilisation of the enzyme also may allow easy recovery of the enzyme after the amidation reaction, so that it can be recycled and repeatedly used in consecutive amidation reactions.

The amount of enzyme used may be chosen within wide limits, depending upon its catalytic activity under reaction conditions, the desired conversion and the desired reaction time. Usually, the amount is at least 0.01 wt. %, at least 0.1 wt. %, at least 1 wt. %, at least 10 wt. % or at least 20 wt. %, based on the weight of the substrate. For practical reasons the amount is usually 1000 wt. % or less, in particular 750 wt. % or less, 500 wt. % or less, 200 wt. % or less, 100 wt. % or less, 50 wt. % or less, or 25 wt. % or less, based on the weight of the substrate.

It is possible to carry out the enzymatic amidation reaction in an inert organic solvent, for instance N,N-dimethylformamide (DMF), N-methyl-pyrrolidinone (NMP), N,N-dimethylacetamide (DMA), dimethylsulphoxide (DMSO), acetonitrile, a hydrocarbon such as toluene, a halogenated hydrocarbon, such as dichloromethane (DCM), 1,2-dichloroethane or chloroform, an ether, such as methyl-t-butyl ether (MTBE), tetrahydrofuran (THF), 2-methyl-tetrahydrofuran (Me-THF) or 1,2-dimethoxyethane, or a (halogenated) alcohol, such as 2,2,2-trifluoroethanol (TFE), *tert-*Butanol (*^{t}*BuOH) or a mixture of these organic solvents. Preferably, the enzymatic amidation reaction may be carried out in an organic solvent or organic solvent mixture comprising MTBE, THF, Me-THF, 1,2-dimethoxyethane, dichloromethane, 1,2-dichloroethane, TFE, DMF, NMP, DMA or DMSO. Most preferably, the enzymatic amidation reaction may be carried out in an organic solvent or organic solvent mixture comprising MTBE, a mixture of MTBE with DMF or NMP or DMA or DMSO, dichloromethane or a mixture of dichloromethane with DMF or NMP or DMA or DMSO.

The enzymatic C-terminal amidation is typically carried out under substantially non-aqueous conditions. As the skilled person will understand, a small amount of water may be desired, depending upon the enzyme, to enable the enzyme to properly perform its catalytic activity.

With substantially non-aqueous is meant that the reaction medium is free of water or contains a minor amount of water, *i.e*. an amount of 1 vol% or less water, based on the total volume of liquids in the reaction medium. The reaction medium may be dispersed in a second liquid phase or another liquid phase may be dispersed in the reaction medium. In case of a dual or multiphase system, the specified water content is based on the volume of liquids in the phase wherein the amidation reaction (at least predominantly) takes place.

A desired upper limit for the water concentration depends on the concentration of the amino acid or peptide ammonium salt, on the specific enzyme, the solvent used, the nature of the amino acid or peptide C-terminal amide to be synthesised (e.g. the size of the peptide and the sequence of the amino acids), the desired final conversion and the desired reaction rate.

Preferably, the water concentration is 1.0 vol% or less, more preferably 0.5 vol% or less, even more preferably 0.1 vol% or less, and most preferably 0.05 vol% or less, whilst still retaining substantial desired enzyme activity.

No lower limit for the water concentration is presented here, because the minimal amount of water that may need to be present is below the detection limits of well known analytical methods, such as Karl-Fischer titration. In an advantageous embodiment, water that is released by the enzyme, may be removed continuously or intermittently. In principle, the water removal may be accomplished in a manner known in the art. Very suitable for the water removal is evaporation, such as azeotropic removal using vacuum or distillation. Good results have in particular been achieved using molecular sieves. The addition of various amounts of molecular sieves to the enzymatic amidation reaction allows the variation of the water concentration below its detection limit. A too low water concentration, for instance obtained by the addition of a large amount of molecular sieves, may in some cases lead to gradual (partial) enzyme deactivation during the amidation reaction. The man skilled in the art can easily determine the optimal water concentration for a certain amidation reaction by variation of the amount of molecular sieves. In case of (partial) enzyme deactivation during the enzymatic amidation reaction, the (partly) deactivated enzyme may be completely or almost completely reactivated by rehydration, for instance by stirring the (partly) deactivated enzyme in an aqueous solution. This reactivation may allow repeated use of the enzyme in consecutive amidation reactions. In some cases, in particular with lyophilized enzymes, the enzyme needs to be hydrated before the enzymatic amidation in order to get sufficient catalytic activity. In case of non-immobilized enzymes such a hydration may be performed by stirring in aqueous solution followed by precipitation, for instance with a water-miscible organic solvent such as t-butanol. In case of immobilized enzymes such a hydration may be performed by washing with an aqueous solution, followed by washing with one or more organic solvents, for instance with a water-miscible organic solvent such as tert-butanol and a water-immiscible solvent such as MTBE.

In principle the pH used in the amidation reaction (in as far as a pH exists in the chosen reaction medium) may be chosen within wide limits, as long as a pH is chosen at which the enzyme shows sufficient activity. Such a pH is usually known for the enzyme to be used and may be based on its known hydrolytic activity in an aqueous solution, or can be routinely determined, making use of a known substrate for the enzyme under known reaction conditions. It may in particular be chosen to be about neutral. If desired, alkaline or acidic conditions may be used, depending on the enzyme. If desired, the pH may be adjusted using an acid and/or a base or the pH may be buffered with a suitable combination of an acid and a base. Suitable acids and bases are in particular those soluble in the reaction medium, e.g. from the group of ammonia and organic solvent-soluble acids, such as acetic acid and formic acid.

In principle the temperature used in the amidation reaction is not critical, as long as the enzyme shows substantial activity. Generally, the temperature may be at least 0 °C, in particular at least 15°C. A desired maximum temperature depends upon the enzyme. In general such maximum temperature is known in the art, *e.g*. indicated in a product data sheet in case of a commercially available enzyme, or can be determined routinely based on common general knowledge and the information disclosed herein. The temperature is usually 70°C or less, in particular 60°C or less or 50°C or less. In particular if a thermophilic hydrolytic enzyme is used, the temperature may be chosen relatively high, for instance in the range of 40 to 90°C.

Optimal temperature conditions can be identified for a specific enzyme by a person skilled in the art through routine experimentation, based on common general knowledge and the information disclosed herein. For instance, for subtilisin, in particular subtilisin A the temperature may advantageously be in the range of 25-60°C.

The invention will now be illustrated by the following examples.

### EXAMPLES

Unless stated otherwise, chemicals were obtained from commercial sources and used without further purification. The 2-chlorotrityl resin was purchased from GL Biochen (China). Savinase, Esperase and Everlase were purchased from Novozymes. Protease from *Bacillus sp.* (three different variants) and Protease from *Bacillus licheniformis* were purchased from Sigma. Alcalase-immozyme was purchased from ChiralVision. ¹H and ¹³C NMR spectra were recorded on a Bruker Avance 300 MHz NMR spectrometer and chemical shifts are given in ppm (δ) relative to TMS (0.00 ppm), DMSO-*d₆* (2.50 ppm for ¹H or 39.9 ppm for ¹³C) or CDCl₃ (77.0 ppm for ¹³C). 3Å and 5Å molecular sieves (8 to 12 mesh, Acros) were activated under reduced pressure at 200°C. *Tert*-butanol (*^{t}*BuOH) was stored on these molecular sieves. *^{t}*BuOH was preheated to a liquid (45°C) before use. Analytical HPLC was performed on an HP1090 Liquid Chromatograph, using a reversed-phase column (Inertsil ODS-3, C18, 5µm, 150 × 4.6 mm) at 40°C. UV detection was performed at 220 nm using a UV-VIS 204 Linear spectrometer. The gradient program was: 0-25 min linear gradient ramp from 5% to 98% eluent B and from 25.1-30 min with 5% eluent B (eluent A: 0.5 mL/L methane sulfonic acid (MSA) in H₂O, eluent B 0.5 mL/L MSA in acetonitrile). The flow was 1 mL/min from 0-25.1 min and 2 mL/min from 25.2-29.8 min, then back to 1 mL/min until stop at 30 min. Injection volumes were 20 µL. LC-MS analysis was performed using the same buffers and gradient programs as for analytical HPLC. Chromatograms were recorded on a Deca XP ion trap LC-MS (ThermoFisher Scientific) using a positive ion electro spray (ESI) in full scan mode: range of 300-2000 amu. The amide product yields of the amidation reactions were estimated by comparing the integrated areas of the product peak with those of the starting material and assuming the same extinction coefficient for the product as for the starting material. Preparative HPLC was performed on a Varian PrepStar system using a stationary-phase column (Pursuit XRs, C18, 10 µm particle size, 500 × 41.4 mm). The gradient program was: 0-25 min linear gradient ramp from 5% to 98% eluent B and from 25.1-30 min with 5% eluent B (eluent A: 0.5 mL/L trifluoroacetic acid (TFA) in H₂O, eluent B 0.5 mL/L TFA in acetonitrile) with a flow of 80 mL/min. Alcalase-CLEA-OM was purchased from CLEA-Technologies (Delft, The Netherlands) and contained 3.5 wt% water. This Alcalase-CLEA-OM was treated as follows before use: 1 g Alcalase-CLEA-OM was suspended in 20 mL ^{t}BuOH and crushed with a spatula. After filtration, this procedure was repeated with 20 mL MTBE. Finally the enzyme was sieved (d = 0.250 mm) to remove large enzyme particles. Liquid Alcalase was treated as follows before use: 10 mL of Alcalase (brown liquid solution, Novozymes type 2.5L DX) was diluted with 20 mL *^{t}*BuOH followed by agitation and subsequent centrifugation (3.500 rpm) of the precipitates and decantation of the supernatant. The pellet was resuspended in 30 mL *^{t}*BuOH followed by agitation, centrifugation (3.500 rpm) and decantation. Alcalase-imibond and Alcalase-epobond were purchased from SPRIN technologies (Trieste, Italy) and were, before use, washed with phosphate buffer (10 mL/g, 100 mM, pH 7.5, 3 x), *^{t}*BuOH (3 x 10mL/g) and MTBE (1x10 mL/g). The same washing procedure was applied, before use, for Alcalase-immozyme. Subtilisin A and Proteinase K lyophilised powders (Sigma) were, before use, treated as follows: 1 gram of lyophilised powder was dissolved in 10 mL phosphate buffer (100 mM, pH 7.5) followed by the addition of 20 mL *^{t}*BuOH followed by agitation and subsequent centrifugation (3.500 rpm) of the precipitates and decantation of the supernatant. The pellet was resuspended in 30 mL *^{t}*BuOH followed by agitation, centrifugation (3.500 rpm) and decantation. This procedure was repeated twice. Peptide *C*-terminal carboxylic acid starting materials were synthesized on a 2-chlorotrityl resin using standard SPPS protocols (see Fmoc Solid Phase Peptide Synthesis by W.C. Chan and P.D. White, Oxford university press, 2004).

### EXAMPLE 1. Amidation of peptides using Alcalase-CLEA-OM.

50 mg of Alcalase-CLEA-OM was added to a mixture of 0.1 mmol peptide and 1.1 equiv 2 N NH₃ in dioxane in 5 mL ^{t}BuOH/DMF (82.5/17.5 v/v) and 100 mg 3Å molecular sieves. This mixture was shaken at 50°C, 150 rpm for 16h and analysed by LC-MS.

| Starting material | Amide product | Conversion to amide product (%) |
|---|---|---|
| Cbz-Phe-Leu-Ala-O-NH4+ | Cbz-Phe-Leu-Ala-NH2 | 94 |
| Cbz-Gly-Gly-Ala-O-NH4+ | Cbz-Gly-Gly-Ala-NH2 | 92 |
| Cbz-Val-Ala-O-NH4+ | Cbz-Val-Ala-NH2 | 91 |
| Cbz-Ile-Met-O-NH4+ | Cbz-Ile-Met-NH2 | 89 |
| Cbz-Gly-Leu-Ala-O-NH4+ | Cbz-Gly-Leu-Ala-NH2 | 89 |
| Cbz-Ala-Leu-O-NH4+ | Cbz-Ala-Leu-NH2 | 87 |
| Cbz-Ala-Phe-O-NH4+ | Cbz-Ala-Phe-NH2 | 87 |
| Cbz-Ala-Ala-O-NH4+ | Cbz-Ala-Ala-NH2 | 86 |
| Cbz-Val-Met-O-NH4+ | Cbz-Val-Met-NH2 | 85 |
| Cbz-Gly-Pro-Ala-O-NH4+ | Cbz-Gly-Pro-Ala-NH2 | 85 |
| Cbz-Gly-Phe-Ala-O-NH4+ | Cbz-Gly-Phe-Ala-NH2 | 82 |
| Cbz-Ala-Met-O-NH4+ | Cbz-Ala-Met-NH2 | 81 |
| Cbz-Tyr-Leu-O-NH4+ | Cbz-Tyr-Leu-NH2 | 81 |
| Cbz-Gly-Phe-O-NH4+ | Cbz-Gly-Phe-NH2 | 81 |
| Cbz-Val-Phe-O-NH4+ | Cbz-Val-Phe-NH2 | 80 |
| Cbz-Leu-Phe-O-NH4+ | Cbz-Leu-Phe-NH2 | 79 |
| Cbz-Gly-Ile-Ala-O-NH4+ | Cbz-Gly-Ile-Ala-NH2 | 78 |
| Cbz-Phe-Leu-O-NH4+ | Cbz-Phe-Leu-NH2 | 77 |
| Cbz-Ala-Pro-Leu-O-NH4+ | Cbz-Ala-Pro-Leu-NH2 | 68 |
| Cbz-Pro-Leu-Gly-O-NH4+ | Cbz-Pro-Leu-Gly-NH2 | 53 |
| Cbz-Gly-Tyr-O-NH4+ | Cbz-Gly-Tyr-NH2 | 46 |
| Cbz-Val-Gly-O-NH4+ | Cbz-Val-Gly-NH2 | 30 |

This table demonstrates that a variety of peptides can be amidated in good yields using Alcalase-CLEA-OM and the peptide ammonium salt.

### EXAMPLE 2. Amidation and purification of Cbz-Val-Ala-OH

100 mg of Alcalase-CLEA-OM was added to a mixture of 0.1 mmol Cbz-Val-Ala-OH and 1.1 equiv 2 N NH₃ in dioxane in 5 mL ^{t}BuOH/DMF (82.5/17.5 v/v) and 100 mg 3Å molecular sieves. This mixture was shaken at 50°C, 150 rpm for 16 h. The reaction mixture was filtrated and the enzyme was resuspended in 5 mL MeOH followed by filtration (3 ×) and in 25 mL EtOAc followed by filtration (3x). The combined organic layers were washed with 50 mL saturated aqueous NaHCO₃, 50 mL brine, dried over Na₂SO₄, concentrated *in vacuo* and dried by co-evaporation with 50 mL of toluene (2×) and 50 mL of CHCl₃ (2×). Cbz-Val-Ala-NH₂ was obtained in a yield of 89%.

| Amide product | NMR data |
|---|---|
| Cbz-Ala-Val-NH2 | 1H NMR (DMSO-d6, 300 MHz): δ = 0.84 (dd, J = 6.9 and 11.4 Hz, 6H), 1.19 (d, J = 7.2 Hz, 3H), 1.95-2.02 (m, 1H), 3.83-3.89 (m, 1H), 4.19-4.25 (m, 1H), 5.03 (s, 2H), 7.00 (s, 1H), 7.32-7.37 (m, 7H), 7.90 (d, J = 7.5 Hz, 1H); 13C NMR (DMSO-d6, 75 MHz): δ = 17.8, 18.3, 19.1, 30.1, 47.7, 60.0, 65.3, 127.5, 127.6, 128.2, 136.9, 156.1, 170.5, 173.9. |

This example shows that the amidated products can be obtained in high yield and purity.

### EXAMPLE 3. Amidation and purification of a peptide sequence with various degrees of protection using Alcalase-CLEA-OM.

100 mg of Alcalase-CLEA-OM was added to a mixture of 0.1 mmol peptide and 2.2 equiv (1.1 for the fully protected peptide) 2 N NH₃ in dioxane in 5 mL ^{t}BuOH/DMF (82.5/17.5 v/v) and 100 mg 3Å molecular sieves. This mixture was shaken at 50°C, 150 rpm for 16 h. The reaction mixture was filtrated and the enzyme was resuspended in 5 mL MeOH followed by filtration (3 ×) and in 5 mL DMF followed by filtration (3x). The combided filtrates were evaporated and the peptide C-terminal amide products were purified by preparative HPLC and lyophilized from acetonitrile/water (1/1, v/v).

| Starting material | Amide product | Yield (%) |
|---|---|---|
| Ac-Trp(Boc)-Met-Asp(tBu)-Phe-O-NH4+ | Ac-Trp(Boc)-Met-Asp(tBu)-Phe-NH2 | 77 |
| H-Trp(Boc)-Met-Asp(tBu)-Phe-O-NH4+ | TFA·H-Trp(Boc)-Met-Asp(tBu)-Phe-NH2 | 68 |
| H-Trp-Met-Asp(O-NH4+)-Phe- O-NH4+ | TFA·H-Trp(TFA)-Met-Asp(O-NH4+)-Phe-NH2 | 63 |

This table demonstrates that *N*- and side-chain protected as well as unprotected peptides can be selectively amidated on the *C*-terminal carboxylic function in good yields using Alcalase-CLEA-OM.

| Amide product | NMR data |
|---|---|
| Ac-Trp(Boc)-Met-Asp(tBu)-Phe-NH2 | 1H NMR (DMSO-d6, 300 MHz): δ = 1.35 (s, 9H), 1.62 (s, 9H), 1.77-1.94 (m, 5H), 2.01 (s, 3H), 2.28-2.45 (m, 2H), 2.61-2.68 (m, 1H), 2.79-3.09 (m, 4H), 4.26-4.41 (m, 2H), 4.50-4.64 (m, 2H), 7.14-7.33 (m, 9H), 7.53 (s, 1H), 7.67 (d, 1H), 7.84 (d, 1H), 8.00 (d, 1H), 8.17-8.24 (m, 3H); 13C NMR (DMSO-d6, 75 MHz): δ = 14.5, 27.6, 29.2, 32.5, 37.2, 49.4, 51.6, 51.8, 53.7, 80.2, 83.6, 113.3, 114.7, 119.4, 122.3, 124.4, 125.6, 126.2, 127.9, 129.0, 129.8, 134.8, 137.6, 148.9, 168.0, 169.2, 169.7, 170.1, 172.4. |
| TFA·H-Trp(Boc)-Met-Asp(tBu)-Phe-NH2 | 1H NMR (DMSO-d6, 300 MHz): δ = 1.36 (s, 9H), 1.63 (s, 9H), 1.68-1.95 (m, 2H), 2.03 (s, 3H), 2.64-2.81 (m, 2H), 2.91-3.05 (m, 2H), 3.11-3.21 (m, 1H), 4.10-4.21 (m, 1H), 4.36-4.48 (m, 2H), 4.55-4.63 (m, 1H), 7.14-7.37 (m, 9H), 7.63 (s, 1H), 7.78 (d, 1H), 7.93 (d, 1H), 8.00-8.10 (m, 5H), 8.40 (d, 1H), 8.90 (d, 1H); 13C NMR (DMSO-d6, 75 MHz): δ = 14.5, 22.4, 26.9, 27.6, 29.3, 31.7, 37.3, 49.5, 52.0, 52.5, 53.8, 80.2, 83.4, 114.5, 116.6, 119.4, 122.3, 123.8, 124.2, 126.1, 128.0, 129.0, 130.2, 134.5, 137.7, 149.0, 169.3, 169.4, 169.7, 170.8, 171.5, 172.6. |
| TFA·H-Trp(TFA)-Met-Asp-Phe-NH2 | 1H NMR (DMSO-d6, 300 MHz): δ = 1.68-1.90 (m, 2H), 1.96 (s, 3H), 2.80-2.92 (m, 2H), 3.01-3.13 (m, 2H), 3.14-3.20 (m, 1H), 3.94-4.05 (m, 1H), 4.28-4.40 (m, 2H), 4.46-4.53 (m, 1H), 6.95-7.32 (m, 11H), 7.62 (d, 1H), 7.88-7.94 (m, 4H), 8.32 (d, 1H), 8.75 (d, 1H), 10.9 (s, 1H), 12.3 (s, 1H); 13C NMR (DMSO-d6, 75 MHz): δ = 14.5, 16.8, 27.3, 29.3, 32.3, 35.9, 36.4, 37.3, 49.5, 51.8, 52.4, 53.8, 106.7, 111.4, 118.4, 121.0, 125.0, 126.1, 127.0, 128.0, 129.0, 136.2, 137.7, 168.4, 167.0, 170.3, 171.9, 172.5. |

### EXAMPLE 4. Amidation of Cbz-Val-Ala-OH using various serine endopeptidases.

100 mg of enzyme was added to a mixture of 0.1 mmol Cbz-Val-Ala-OH and 1.1 equiv 2 N NH₃ in dioxane in 5 mL *^{t}*BuOH/DMF (82.5/17.5 v/v) and 100 mg 3Å molecular sieves. The mixtures were shaken at 50°C, 150 rpm for 16 h and analysed by HPLC. Good conversion to Cbz-Val-Ala-NH₂ (>50%) was obtained using various serine endopeptidases and using Alcalase immobilized in various ways: Alcalase-CLEA-OM, Alcalase-imibond, Alcalase-epobond, Alcalase-immozyme, Liquid Alcalase, Subtilisine A, Proteinase K, Savinase, Esperase, Everlase, Protease from *Bacillus sp.* (three available variants) and Protease from *Bacillus licheniformis.*

### EXAMPLE 5. Amidation of Cbz-Phe-OH using various solvents.

1 g of Cbz-Phe-OH was dissolved in 10 mL MTBE followed by bubbling of NH₃ gas through this solution for 30 sec. The precipitated Cbz-Phe-O-NH₄⁺ was filtered off and dried *in vacuo.* 100 mg of Alcalase-CLEA-OM was added to a mixture of 0.1 mmol Cbz-Phe-O-NH₄⁺, 5 mL solvent and 100 mg 3Å molecular sieves. The mixtures were shaken at 50°C, 150 rpm for 16 h and analysed by HPLC. Good conversions to Cbz-Phe-NH₂ (>50%) were obtained using the following solvent(s) (mixtures): *^{t}*BuOH/DMF (82.5/17.5 v/v), *^{t}*BuOH, THF, Me-THF, MTBE, DMF/MTBE (1/9, v/v), DMF/THF (1/9, v/v) toluene, DCM, dioxane and acetonitrile.

### EXAMPLE 6. Amidation using a separate ammonium source compared to amidation using the ammonium salt of the peptide.

Peptide ammonium salt: 50 mg of Alcalase-CLEA-OM was added to a mixture of 0.1 mmol Cbz-Phe-Leu-Ala-O-NH₄⁺ in 5 mL ^{t}BuOH/DMF (82.5/17.5 v/v) and 100 mg 3Å molecular sieves. This mixture was shaken at 50°C, 150 rpm for 16h. Composition of the reaction mixture according to LC-MS analysis: Cbz-Phe-Leu-Ala-O-NH₄⁺ (5%, starting material), Cbz-Phe-Leu-Ala-NH₂ (94%, product) and Cbz-Phe-Leu-NH₂ (1%, transamidation byproduct).

Separate ammonium salt: 50 mg of Alcalase-CLEA-OM was added to a mixture of 0.1 mmol Cbz-Phe-Leu-Ala-OH in 5 mL *^{t}*BuOH/DMF (82.5/17.5 v/v), 1 mmol ammonium carbamate. This mixture was shaken at 50°C, 150 rpm for 16h. Composition of the reaction mixture according to LC-MS analysis: Cbz-Phe-Leu-Ala-OH (4%, starting material), Cbz-Phe-Leu-Ala-NH₂ (81%, product), Cbz-Phe-Leu-N H2 (9%, transamidation byproduct), Cbz-Phe-Leu-OH (4%, peptide bond hydrolysis byproduct) and Cbz-Phe-NH₂ (2%, transamidation byproduct).

These experiments show that when using a separate ammonium salt less product is obtained and more side reactions occur.

### EXAMPLE 7. Preparation of a peptide ammonium salt

Using ammonia gas: 0.5 mmol Cbz-Phe-Leu-Ala-OH was dissolved in 5 mL MTBE and NH₃ gas was bubbled through this solution for 30 seconds. The precipitated Cbz-Phe-Leu-Ala-O⁻NH₄⁺ was filtered off and dried *in vacuo.*

Using ammonia in solution: 0.5 mmol Cbz-Phe-Leu-Ala-OH was dissolved in 5 mL MTBE and to this solution 1.1 equivalent of 2N NH₃ in dioxane was added. The precipitated Cbz-Phe-Leu-Ala-O-NH₄⁺ was filtered off and dried *in vacuo.*

## Claims

1. Method for enzymatically synthesising an amino acid or peptide C-terminal amide, from the corresponding N-protected amino acid ammonium salt or optionally N-protected peptide C-terminal carboxylic acid ammonium salt, by contacting said N-protected amino acid ammonium salt or optionally N-protected peptide C-terminal carboxylic acid ammonium salt with a serine endopeptidase in an organic solvent or an organic solvent mixture containing 1 vol% or less water.

2. Method according to claim 1, wherein the amino acid or peptide C-terminal carboxylic acid ammonium salt has been prepared by adding gaseous NH₃ or an NH₃ solution in an organic solvent or organic solvent mixture to a solution of the amino acid or peptide C-terminal carboxylic acid.

3. Method according to any of claims 1 or 2, wherein the serine endopeptidase is a subtilisin or a mutant thereof.

4. Method according to any of claims 1 to 3, wherein the enzyme is in an immobilised form.

5. Method according to claims 4, wherein the immobilized enzyme is in the form of a cross-linked enzyme aggregate (CLEA).

6. Method according to any of claims 1 to 5, wherein the organic solvent or organic solvent mixture comprises MTBE, THF, Me-THF, 1,2-dimethoxyethane, dichloromethane, 1,2-dichloroethane, TFE, DMF, NMP, DMA , *t*BuOH or DMSO.

7. Method according to claim 6, wherein the organic solvent or organic solvent mixture comprises MTBE, a mixture of MTBE with DMF or NMP or DMA or DMSO, dichloromethane or a mixture of dichloromethane with DMF or NMP or DMA or DMSO.

8. Method according to any of claims 1 to 7, wherein the organic solvent or the organic solvent mixture contains 0.5 vol% or less water, preferably 0.1 vol% or less.

9. Method according to any of claims 1 to 8, wherein water that is released by the enzyme is removed continuously or intermittently.

10. Method according to any of claims 1 to 9, wherein the water that is released by the enzyme is removed using molecular sieves.
